# EUROPEAN PATENT APPLICATION

(11) **EP 4 529 832 A2**
(43) Date of publication of application: **02.04.2025**
(21) Application number: 25156480.3
(22) Date of filing: 26.12.2019
(51) Int. Cl.: A61B 5/00

(54) **SENSOR DELIVERY SYSTEM**

(30) Priority: 26.12.2018 US 201862784887 P; 09.01.2019 US 201916243183
(62) Divisional of application: 19845871.3
(71) Applicant: Endotronix, Inc., Lisle, IL 60532 (US)
(72) Inventor: CHRONOS, Nicholas, Lisle, 60532 (US); NAGY, Michael, Lisle, 60532 (US); ROWLAND, Harry, Lisle, 60532 (US); PANIAN, Tyler, Lisle, 60532 (US); WILSCHKE, Thomas, Lisle, 60532 (US); ROYER, Trace, Lisle, 60532 (US); COYLE, James, Lisle, 60532 (US); MAHR, David, Lisle, 60532 (US); FOROUZAN, Omid, Lisle, 60532 (US); POFF DVM, Brad, Lisle, 60532 (US)
(74) Representative: Müller-Boré & Partner Patentanwälte PartG mbB

(57) **Abstract**

An implant delivery system (200) comprising: an implant (101) configured to measure pressure and to wirelessly communicate information to a receiver or reader device; a first sheath (202) and a second sheath (204), wherein said first sheath (202) is positioned at least partially within said second sheath (204), the first sheath (202) is translatable relative to said second sheath (204) and wherein the first sheath (202) comprises an exterior circumference; wherein said implant (101) is directly connected to the exterior circumference of said first sheath (202) and is deployable from the exterior circumference, and
wherein said first sheath (202) and said second sheath (204) are movable with respect to one another to deploy and release said implant (101) to a target site in an anatomy; and further wherein said first sheath (202) is configured to be partially inserted into a living body such that a proximal end of said second sheath (204) remains external to said living body and a distal end of said first sheath (202) is internal to said living body.

## Description

### CROSS-REFERENCE TO RELATED APPLICATIONS

This application claims priority to U.S. Provisional Patent App. No. 62/784,887 entitled "SENSOR DELIVERY SYSTEM AND METHOD" and filed December 26, 2018. This application is a continuation-in-part of U.S. Patent App. No. 16/243183 entitled "PRESSURE SENSOR, ANCHOR, DELIVERY SYSTEM AND METHOD and filed January 9, 2019 which claims priority to U.S. Patent No. 10,206,592 entitled "PRESSURE SENSOR, ANCHOR, DELIVERY SYSTEM AND METHOD and filed March 16, 2015 which claims priority to PCT Patent App. No. PCT/US2013/059769 entitled "PRESSURE SENSOR, ANCHOR, DELIVERY SYSTEM AND METHOD and filed on September 13, 2013, that claims priority to U.S. Provisional Patent App. No. 61/701,058 entitled "PRESSURE SENSOR, ANCHOR, DELIVERY SYSTEM AND METHOD" and filed on September 14, 2012, and further claims priority to PCT Patent App. No. PCT/US2011/045583 entitled "PRESSURE SENSOR, CENTERING ANCHOR, DELIVERY SYSTEM AND METHOD" and filed on July 27, 2011. This application is also a continuation-in-part of U.S. Patent App. No. 15/213,712 entitled "PRESSURE SENSING IMPLANT," filed on July 19, 2016 which is a continuation-in-part of U.S. Patent Application No. 14/777,654 entitled "PRESSURE SENSING IMPLANT" filed on September 16, 2015 each of which are incorporated by reference.

### FIELD OF INVENTION

This application relates to a medical implantable devices, positioning and anchoring mechanisms, delivery systems and more particularly to a method for delivering and positioning medical implantable devices into the human body.

### BACKGROUND

Delivery systems and positioning and anchoring devices are currently being used in medical procedures to guide and position devices from a remote site to a target site within a body. Catheter-based delivery systems are generally used to guide and position invasive implantables, such as pressure sensors, within the cardiovascular system of a patient. There exist various commercial implantable pressure sensors that are permanently implanted within the cardiovascular system using catheter-based delivery systems. One example is the CardioMEMS device by Abbott Labs, Inc. References: "CardioMEMS HF System, PA Sensor and Delivery System", St Jude Medical Publication US-2000054 B EN (06/14); Shavelle, D, MD; Jermyn, R, MD; "The CardioMEMS Heart Failure Sensor: A Procedural Guide for Implanting Physicians", Journal of Invasive Cardiology 2016; 28(7):273-279.; "CardioMEMS HF System, PA Sensor and Delivery System, Model CM2000, User's Manual", CardioMEMS publication LA-400275-03, Art 60056412, May 2014; "CardioMEMS Implant Procedure and Patient Training Video" St Jude Medical video SJM-MEM-0215-0085a. 2015.

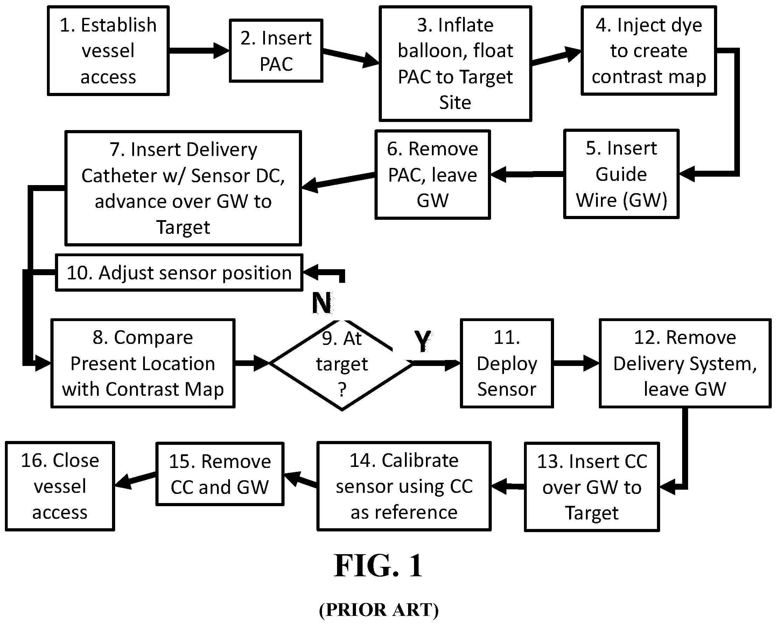

Figure 1 illustrates a schematic diagram of a prior art catheter-based delivery system disclosed by the described publications that describes a method for implanting a pressure sensor implant within the pulmonary artery of a patient using a pulmonary artery catheter ("PAC"). Here. the PAC includes a catheter having an inflatable balloon along a distal end of an elongated tube structure. The PAC is configured to provide fluid communication from a target site to the proximal end of the catheter outside the body. The PAC allows for pressure measurements as well as drug injections. It may also have other features, such as ability to measure cardiac output using the well-known thermodilution method. In a known embodiment, the target site includes the pulmonary artery but may be any other location within a patient's anatomy, preferably within the cardiovascular system.

The steps disclosed by this procedure include the following as illustrated by Figure 1. Venous access may be established at an access location along the patient's anatomy by puncturing a vein percutaneously (1). This may also include inserting an "introducer" at the access location to support the insertion of various catheters. The femoral vein is a typical access location; jugular, brachial, and subclavian vein are also known access locations. The distal end of a first pulmonary artery catheter ("PAC") is then inserted at the access location (2). A balloon may be inflated along a distal portion or distal top of the PAC within the vascularity of the patient (3). The inflated balloon may be used to free up blocked vessels or temporarily block blood flow to a certain branch. It may also be used to float the PAC along with the bloodstream towards the target anatomy. The target site may be anything in the path of venous flow, including any of the veins, the heart, the pulmonary artery, or even inside the gastrointestinal (GI) tract. The PAC may also be configured to take pressure readings or measure output (via thermodilution) along its journey through the anatomy up to and including the target site. Thremodilution is a method of measuring blood flow based on the premise that when an indicator substance is added to circulating blood, the rate of blood flow is inversely proportional to the indicator concentration change over time. The measurements taken of the target site, for example, could include but is not limited to the right atrial pressure, right ventricular pressure, pulmonary artery pressure, and pulmonary artery wedge pressure.

Contrast dye may be injected through a port in the PAC to assist with imaging and examining the anatomy with a fluoroscope (4). The contrast dye may be injected into the anatomy by introducing the contrast dye through the proximal end of the PAC outside the body, to be dispensed near the distal end of the PAC within the body. Imaging display may then clearly illustrate the vasculature with the contrast dye. This process is called angiography and the image you get from it is an angiogram. Prior art systems utilize static photos / freeze-frames of the angiogram to assist with positioning the sensor or implant with a second catheter as described below.

Preferably, during this procedure, the distal end of the PAC may be located at or near the target site and a guide wire (GW) may be inserted or positioned within a lumen of the PAC (5). The guide wire (GW) may slide or otherwise be withdrawn from a lumen within the PAC to be positioned at or adjacent the target site (6). The PAC may be removed allowing the guide wire (GW) to remain positioned therein. The guide wire (GW) may remain located within the vasculature between the access location and the target site to guide other catheters in and out to the target site (7).

A delivery catheter (DC) including a sensor or implant attached at or near a distal end thereto may be inserted through the access location of the patient with the assistance of the guide wire (GW) to positon the sensor or implant towards the target site. The fluoroscope may visually assist the medical practitioner to monitor the anatomy as the sensor or implant is positioned at or near the target site. The sensor may include fluoro markers to allow the visual assistance of the sensor or implant on the fluoroscope as well as guide wire (GW). However, at this point in the procedure of known delivery systems, contrast dye is not available and live images of the position of the sensor/implant along the DC relative to the vasculature is therefore not available (8). The medical practitioner must utilized the angiogram image acquired when contrast dye was introduced by the PAC moments ago to compare the relative position of the DC and sensor relative to anatomical markers (e.g., ribs, pacemaker, spine). This comparison of step (8) allows the medical practitioner to estimate sizes and locations using known features available from a static image of the angiogram in the estimation of placing the sensor or implant at the target site in the anatomy.

This requires the medical practitioner in control of the delivery catheter to stop and hold its position as the comparison occurs between the live fluoroscopic image (of the sensor) with the static angiogram "map" made moments ago. Steps (9) and (10) reflect the continual iterative process of moving or adjusting the delivery catheter and comparing the angiogram "map" with the live fluoro image until the medical practitioner in control of the deliver catheter estimates that the sensor or implant is positioned at the "estimated" target site. Once reached, the sensor or implant is deployed at the estimated target site (11). This may occur by triggering a mechanism at the proximal end of the delivery catheter that actuates release wires attached to the sensor or implant to release the senor or implant therefrom. The sensor is now deployed and the delivery catheter is removed from the patient (12) as the guide wire remains therein.

A second calibration catheter ("CC"), similar in type to the first pulmonary artery catheter PAC, may be inserted at access location along the guide wire (13). The second calibration catheter CC may include a fluid lumen therein but may not include a distally positioned balloon. Notably, each of these types of catheters are designed for a single use and may be costly. The fluid lumen may extend along the length of the second catheter CC and couple the proximal end to a second sensor. The second sensor may be an off-the-shelf one-use pressure sensor or transducer device that is used to obtain a reference reading (14). The second sensor may be placed at or near the target site that includes the sensor or implant for taking the reference reading. That reference reading may be used to adjust, correct, or otherwise calibrate the sensor or implant as it is placed at the target site. This is "in situ calibration" of the implanted sensor may be performed mathematically in software. The second catheter CC and guide wire GW may be removed (15) and the access location or vessel site may be closed (16).

Even though such implant procedures are minimally invasive and have a good safety record, any surgical procedure causes risk to the patient. There is a desire to minimize the time a patient is under anesthesia and exposed to risk of infection, also to minimize procedure time, use of medical resources, like a catheterization lab and implanting surgical teams. There is also a desire to reduce cost of healthcare by minimizing the amount of disposable and non-disposable equipment required for a given procedure. There is a need to improve the simplicity and efficiency of similar systems and methods for convenience of the medical practitioners and to enhance patient safety.

### SUMMARY

Provided are various embodiments of improvements made to methods, systems and assemblies of implant delivery systems and the associated implants. In one embodiment, provided is an implant delivery system comprising an implant, a first sheath and a second sheath each extending from a proximal end of said implant delivery system, wherein at least said first sheath extends to a distal end of said implant delivery system, wherein said first sheath is positioned at least partially within said second sheath, the first sheath is translatable relative to said second sheath wherein said implant is connected to an exterior surface of said first sheath, and wherein said first sheath and said second sheath are movable with respect to one another to deploy said implant to a target site in an anatomy. Said delivery system may be configured to be partially inserted into a blood vessel of a human body such that said proximal end remains external to said body and said distal end is internal to said body. At least one fluid port may be positioned along a proximal end of said first sheath or second sheath, said fluid port fluidly coupled to a lumen extending down the length of said first sheath or second sheath to allow fluid flow through said lumen. Fluid may be injected through said port and may includes one of: a drug; a fluid used to enhance anatomical imaging; fluoroscopic contrast dye; barium; a radioactive material; blood; plasma; saline solution; a blood component; a particle suspension; a nano-device; and a nanomaterial. The at least one fluid port may further be configured to operatively couple to a device located outside of said body wherein said device is a pressure transducer, configured to measure a fluid pressure at the distal end of said first or said second sheath. Said measurement of fluid pressure at the distal end of said first or second sheath may be used to calibrate or assess the accuracy of said implant.

At least one marker may be placed on the delivery system that is configured to be visible with a fluoroscope. Said marker may include a radio opaque material positioned on at least one of: a distal tip of said first sheath; the distal portion of said second sheath; a portion of said implant; and as a plurality of lines spaced along a portion of said first or said second sheath. The marker may be attached to at least one anchor on the implant. A plurality of markers may be positioned along said implant in an asymmetric pattern, said pattern is configured to facilitate determination of implant orientation when viewed on a fluoroscope. Said asymmetric pattern comprises markers at three of the four corners of a two-dimensional rectangle when viewed normal to the plane of said rectangle on a fluoroscope.

Said fluid port may be further configured to allow removal of fluid from said body. A balloon member may be positioned along the delivery system, wherein said balloon member is configured to be inflated to guide said deliver system to a target site. Said balloon member may be configured to facilitate a wedge pressure measurement. Said balloon member may be configured to limit blood flow in a vessel to facilitate implant deployment or retraction. Said balloon member may be configured to hold said first sheath in place with respect to a blood vessel while said implant is released from said first sheath at said target site. A temperature sensor may be placed on said distal end to facilitate measurement of flow rate by thermodilution, wherein said flow rate measurement is configured to determine a cardiac output.

Said second sheath may be configured to allow insertion of a catheter device, wherein said catheter device includes at least one of: a camera, a pressure sensing catheter, a stent placement device, a valve placement device, a microphone, an ablation device, a balloon device, a Swan Ganz catheter, an electrical stimulation device, an ultrasound device, a drug delivery device, a catheter for gripping implanted devices, a catheter for readjusting the position of implanted devices, a catheter for removing implanted devices. Said second sheath may be configured to allow insertion of a catheter device configured to selectively attach to said implant and move the implant proximally when retracted, wherein at least one anchor of said implant is collapsible, and wherein said anchor of said implant is configured to be placed in a collapsed state when said catheter device moves the implant proximally into said second sheath. Said second sheath may be further configured to allow said first sheath to be retracted into said second sheath while said implant is still connected to said first sheath, and further configured to cover said first sheath and said implant while said delivery system is retracted and withdrawn from said body. At least one release wire may extend from said proximal end of said first sheath to said distal end, wherein said release wire is configured to connect said implant to said first sheath. Said implant may be provided with at least one collapsible anchor, and further wherein said at least one release wire connects said implant to said first sheath by exiting at least one slot positioned along said first sheath, crossing over said anchor in said collapsed state, and entering the at least one slot or a second slot along said first sheath. Retraction of said at least one wire proximally may cause said implant to be released from said first sheath.

A reference sensor may be positioned along the first sheath or the second sheath, wherein the reference sensor is independent of said implant. Said reference sensor may be at least one of a: pressure sensor, a blood oxygen level sensor, a microphone, a sensor of tissue optical properties, a temperature sensor, a flow rate sensor, and a chemical sensor. Said system may be configured to allow a user to mechanically couple or decouple said first sheath and said second sheath, such that said sheaths can be made movable or non-movable with respect to one another during use. Said first sheath may be configured with a distal tip made of a soft material to minimize vessel trauma during use, wherein said distal tip has a durometer softer than Shore 40A.

In another embodiment, provided is a method for implanting an implant device in the vasculature of a human body, said method comprising the steps of: establishing access to the vasculature at an access location along the patient's anatomy; inserting a first catheter configured to translate from the access location to a target site within the vasculature; placing a guide wire between said access point to said target location; removing said first catheter while leaving said guide wire in place; inserting a delivery system over said guide wire, said delivery system comprising a first sheath and a second sheath each extending from a proximal end of said implant delivery system, wherein at least said first sheath extends to a distal end of said implant delivery system and wherein said first sheath is positioned at least partially within said second sheath, and includes an implant attached to said first sheath, wherein said first sheath or second sheath configured to allow the injection of contrast dye for angiographic imaging; advancing said delivery system to said target site while using angiographic imaging to position said implant at said target site; deploying said implant from said delivery system at said target site; and withdrawing said second catheter and said guidewire from said body while said medical device remains at said target location. Said delivery system may include a port configured to fluidically couple a portion of said first sheath or second sheath that is inside said vasculature to a portion of said first sheath or second sheath that is outside of said vasculature, said method further comprising the steps of: connecting said outside portion of said first sheath or second sheath to a pressure measurement device; measuring pressure at said inside portion of said first sheath or second sheath using said pressure measurement device; measuring pressure at said inside portion with said implant; comparing said measurement from said pressure measurement device to a measurement made by said implant. The method may further comprise the steps of assessing an accuracy of said implant and calibrating said implant.

Said port may be configured to inject contrast dye within the vasculature. Said implant may be connected to an exterior surface of said first sheath and positioned near a distal end of said second sheath, and wherein said first sheath and said second sheath are movable with respect to one another to deploy said implant to the target site. Said first catheter device comprises one of a balloon member configured to follow vascular flow to translate from said access location to said target site and a steerable tip catheter.

Also provided is a method for implanting an implant in a vasculature of a body, said method comprising the steps of: establishing access to the vasculature at an access location; inserting a catheter device into said vasculature at said access location, said catheter device fitted with said implant and configured to advance to a target site; advancing said catheter device to said target site; deploying said implant from said catheter device at said target site; and withdrawing said catheter device from said body while said implant remains at said target site. Said catheter device may be configured to advance to said target site by a balloon member configured to follow vascular flow to translate from said access location to said target site and a steerable tip catheter. Said catheter device may be configured to inject contrast dye into said vasculature to facilitate angiographic imaging, said method further comprising the step of advancing said catheter to said target location while using angiographic imaging. Said catheter device may be configured to fluidically couple a portion of said catheter device inside said vasculature to a portion of said catheter device that is outside of said vasculature, said method further comprising the steps of: connecting said of said catheter device that is outside of said vasculature to a pressure measurement device; before withdrawing said catheter device from said body, measuring pressure at said portion of said catheter device inside said vasculature using said pressure measurement device; measuring pressure using said implant; comparing said measurement from said pressure measurement device to a measurement made by said implant.

Said catheter device comprises a first sheath and a second sheath each extending from a proximal end of said catheter device, wherein at least said first sheath extends to a distal end of said implant delivery system, wherein said first sheath is positioned at least partially within said second sheath; wherein said implant is connected to an exterior surface of said first sheath and positioned near a distal end of said second sheath, and wherein said first sheath and said second sheath are movable with respect to one another to deploy said implant to a desired target location.

Also provided is an implant delivery system comprising: an implant; a first sheath and a second sheath each extending from a proximal end of said implant delivery system, wherein at least said first sheath extends to a distal end of said implant delivery system, wherein said first sheath is positioned at least partially within said second sheath; wherein said implant is connected to an exterior surface of said first sheath and positioned near a distal end of said second sheath; and wherein said first sheath and second sheath are moveable with respect to one another to deploy said implant to a desired location. Said implant further comprises a rigid housing, and at least one collapsible implant anchor, said at least one anchor being attached to said housing. Said anchor may be comprised of at least one wire that passes through at least one hole that extends through the thickness of said housing; and an enlarged portion of the wire is placed along said wire to retain the wire within the hole. Said hole may be counter-bored, said counter-bored hole comprising a first size hole and a second size hole, wherein the first size is smaller than the second size, wherein the second hole extends only partially through said housing, and further wherein said enlarged portion is configured to fit into said second hole. Each end of said anchors may be attached through a hole in said housing, such that said anchor forms a partial or complete loop including two ends that terminate at points on said housing. Said implant includes a marker configured to be at least partially visible under fluoroscopic imaging. Said marker may be positioned within said hole and includes a radio opaque material. Said anchor wire includes a nitinol material with a platinum core. Said marker may be a radio opaque marker that includes one of a: paint, ink, or preformed cylindrical tube. The marker may be a tube attached to said anchor wire by one of: adhesive, heat shrinking, and friction fit. A plurality of said markers may be spaced at known intervals along said anchor wire or said catheter device to facilitate distance estimation during angiographic imaging. Said marker may be located on the anchor wire near a point where said anchor wire attaches to said housing. Said hole may be filled with a filler material to prevent rotational or translational movement of said anchor relative to said hole, wherein said filler material is selected from: adhesive, potting material, epoxy, silicone, or polymer. A plurality of said markers comprise an asymmetric pattern on said implant body, said pattern configured to facilitate determination of implant orientation when viewed on a fluoroscope. Said asymmetric pattern comprises three marks positioned along three corners of a two-dimensional rectangle when viewed on a fluoroscope.

Further, provided is a method for attaching a wire anchor to a rigid implant body, comprising the steps of: providing at least one hole that extends through an implant housing; passing an anchor wire through said hole; enlarging at least one portion along said wire to prevent said enlarged portion from passing through said hole. Said hole may be formed by counter-boring to create a large portion and a small portion. Said enlarged portion of said wire may be placed into said large portion of said hole. At least one preformed fluoroscopic marker may be placed over said anchor wire prior to enlarging said dimension of said terminal end. Two ends of said wire anchor may be attached to said implant body such that said anchor forms a loop. Said hole may be filled with a filler material to event translational or rotational movement of said anchor.

### DESCRIPTION OF THE DRAWINGS

These, as well as other objects and advantages of this application, will be more completely understood and appreciated by referring to the following more detailed description of the presently preferred exemplary embodiments of the application in conjunction with the accompanying drawings, of which:
Figure 1 illustrates the schematic diagram of a prior art procedure for deploying a sensor to target site within a patient's anatomy;
Figure 2 illustrates an embodiment of an implant attached to a delivery system according to aspects of the instant application;
Figure 3 illustrates the schematic diagram of an embodiment of a method and system according to aspects of the instant application for deploying a sensor to target site within a patient's anatomy;
Figure 4 illustrates the schematic diagram of another embodiment of a method and system according to aspects of the instant application for deploying a sensor to a target site within a patient's anatomy;
Figure 5 illustrates the schematic diagram of another embodiment of a method and system according to aspects of the instant application for deploying a sensor to a target site within a patient's anatomy;
Figure 6A illustrates an embodiment of an implant from Figure 22 of commonly owned US Patent No 10,206,592;
Figure 6B is a cross sectional view of attachment portions of an implant according to aspects of the instant application;
Figure 7A is a cross sectional view of a similar implant to Figure 6B that includes an anchor wire as it is being assembled to the implant according to according to aspects of the instant application;
Figure 7B is a cross sectional view of an embodiment of the implant of Figure 7A that includes the anchor wire with a marker band as it is being assembled to the implant according to according to aspects of the instant application;
Figure 7C is a cross sectional view of an embodiment of the implant of Figure 7A that includes the anchor wire with the marker band as it is being assembled to the implant according to according to aspects of the instant application;
Figure 7D is a cross sectional view of an embodiment of the implant of Figure 7A with the anchor wire and marker band as it is being assembled to the implant according to according to aspects of the instant application;
Figure 7E is a cross sectional view of an embodiment of the implant of Figure 7A with the anchor wire and marker band assembled to the implant according to according to aspects of the instant application;
Figure 8A illustrates a schematic plan view of an embodiment of the implant that includes marker band locations according to aspects of the instant application;
Figure 8B illustrates a schematic plan view of an embodiment of the implant that includes marker band locations according to aspects of the instant application;
Figure 8C illustrates a schematic plan view of an embodi9ment of the implant that includes marker band locations according to aspects of the instant application;
Figure 9 is a cross sectional schematic view of an implant attached to a delivery system according to aspects of the instant application; and
Figure 10 illustrates an embodiment of an implant attached to a retrieval system according to aspects of the instant application.

### DETAILED DESCRIPTION

Reference will now be made in detail to exemplary embodiments of the present teachings, examples of which are illustrated in the accompanying drawings. It is to be understood that other embodiments may be utilized and structural and functional changes may be made without departing from the respective scope of the present teachings. Moreover, features of the various embodiments may be combined or altered without departing from the scope of the present teachings. As such, the following description is presented by way of illustration only and should not limit in any way the various alternatives and modifications that may be made to the illustrated embodiments and still be within the spirit and scope of the present teachings. In this disclosure, any identification of specific shapes, materials, techniques, arrangements, etc. are either related to a specific example presented or are merely a general description of such a shape, material, technique, arrangement, etc.

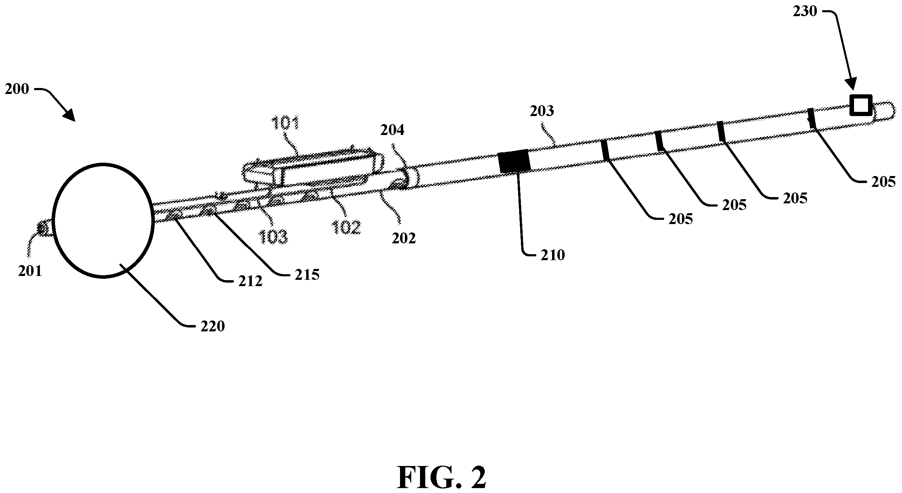

The instant application is directed to a method and system of utilizing a version of the delivery device similar to that disclosed by US Patent US Patent No. 10,206,592 of which this claims priority from. Referring now to the Figures, wherein common elements are identified by the same numbers, Figure 2 illustrates a perspective view of a portion of such an improved delivery device and system 200. The instant application discloses a catheter that includes a first or inner/carrier sheath 202 with a distal tip 201 and a second or outer/torque sheath 204 that extends proximally therefrom. The sheaths include one or more lumens that extend therein to perform a variety of functions including but not limited to: support a guide wire, allow for fluid passage, support release wires, and inflate or deflate a balloon positioned along the length of the delivery device. The first and second sheaths 202, 204 may be elongated and be attached at a proximal end to a handle (not shown) to allow for the control of rotation and translation relative to one another. The carrier sheath 202 and torque sheath 204 may be temporarily fixed to each other such that fixed rotation and translation occurs when desired, but when not desired, carrier sheath 202 and torque sheath 204 may move relative to one another.

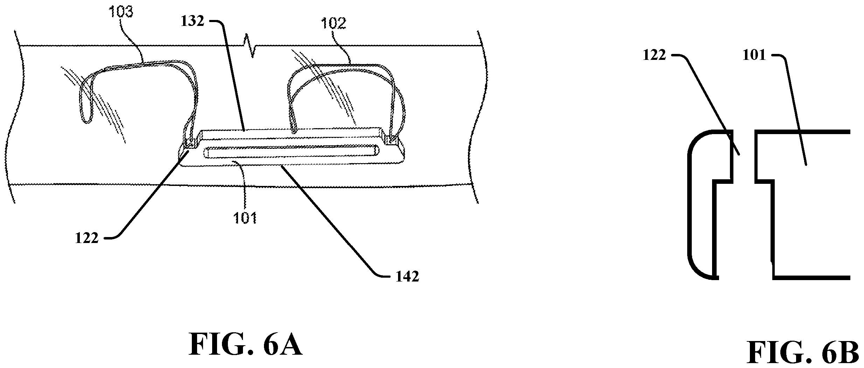

One or more anchor release wires 212 may extend within lumens within the sheaths 202, 204. The wires 212 may extend from a proximal end of the catheter towards the distal end and are configured to temporarily support and attach an implant 101 to the carrier sheath 202. This attachment may be established by engagement of anchors that extend from the implant 101. In one embodiment, the implant may be a wireless device configured to electronically communicate with a receiver or external reader device. The implant may include an LC resonant tank within a housing. In another embodiment, the implant may be a passive device or an active device and include a battery or other power source. The anchors may include a distal anchor 103 and proximal anchor 102 that extend from opposing portions of the implant 101. The anchor release wires 212 attach to the carrier sheath 202 and anchor 102, 103 by entering into and out of one or more slots 215 positioned along the carrier sheath 202. This arrangement may allow the anchors to be collapsed during implantation and to expand (e.g., Figure 6A) once the release wires 212 have been removed.

The carrier sheath 202 may be configured to rotate and to translate with the torque sheath 204 or may be able to rotate and translate relative to the torque sheath 204. This allows the sheaths to move proximal/distal relative to one another or to prevent rotation of one sheath relative to the other. Further, the distal tip 201 as well as desired portions along the sheaths 202, 204 may be covered with radio opaque material to improve visibility during fluoroscopy. Likewise a ruled set of radio opaque markers 205 may be placed along a portion of the sheaths 202, 204 having pre-defined separation distances. These markers 205 may assist with establishing visible cues to assist the medical personnel with placement of the delivery system 200 within the anatomy.

The outer sheath 204 may carry out various functions that are different from prior versions of known systems. These functions include (a) injection of contrast dye for real-time angiography at any time when the outer sheath 204 is in the body; (b) use the outer sheath as the fluid column (aka 'fluid channel' aka 'lumen') that fluidically couples the distal end of the outer sheath to a proximal port 230, positioned outside the patient's body, (c) a pressure transducer (not shown) may be fluidically attached to the proximal port for procuring reference measurements, and (d) a reference sensor 210 may be attached to a distal portion of the outer sheath 204 for procuring reference measurements. Additionally, a balloon member 220 may optionally be positioned along the length of the inner sheath 202 or outer sheath 204 which may be toggled to inflate or deflate to assist with guiding the delivery device within the vasculature as described below. Said balloon member may be configured to be inflated to guide said deliver system to the target site. Further, said balloon member may be configured to facilitate a wedge pressure measurement. Said balloon member may also be configured to I limit blood flow in a vessel to facilitate implant deployment, retraction, or rotation while it may also be configured to hold said first sheath in place with respect to a blood vessel while said implant is released from said first sheath at said target site. Said fluid port may be configured to allow removal of fluid from said body. At least one temperature sensor may be positioned on said distal end/ tip 201 to facilitate measurement of flow rate by thermodilution, wherein said flow rate measurement is configured to determine a cardiac output. Said second sheath may be configured to allow insertion of a catheter device, wherein said catheter device includes at least one of: a camera, a pressure sensing catheter, a stent placement device, a valve placement device, a microphone, an ablation device, a balloon device, a Swan Ganz catheter, an electrical stimulation device, an ultrasound device, a drug delivery device, a catheter for gripping implanted devices, a catheter for readjusting the position of implanted devices, a catheter for removing implanted devices.

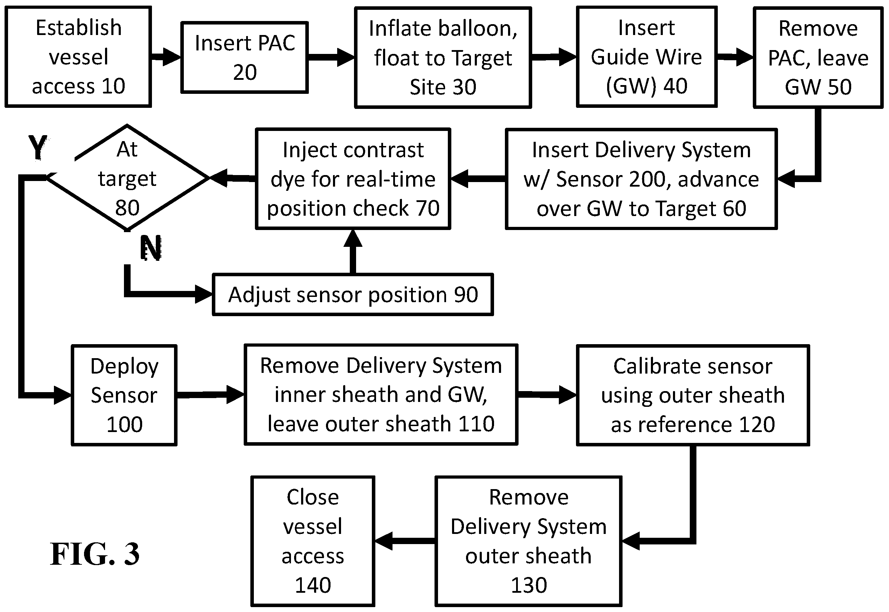

Figure 3 illustrates a schematic diagram of an embodiment of a method and system according to aspects of the instant application for deploying a sensor or implant to a target site within a patient's anatomy. This disclosure contemplates the use of the delivery system 200 for surgically implanting the implant 101 or sensor within the vascularity of a patient to communicate with a reader device (not shown). Various embodiments of the implant/sensor and reader device are contemplated by the following commonly owned patent documents: U.S. Patent App. No. 15/958,613 entitled "ANCHORING SYSTEM FOR A CATHETER DELIVERED DEVICE," filed on April 20, 2018; U.S. Patent App. No. 15/213,712 entitled "PRESSURE SENSING IMPLANT," filed on July 19, 2016; U.S. Patent Application No. 14/777,654 entitled "PRESSURE SENSING IMPLANT" filed on September 16, 2015; U.S. Patent No. 8,493,187 entitled "WIRELESS SENSOR READER" filed on March 19, 2010; U.S. Patent No. 8,899,582 entitled "CARDIAC PRESSURE MONITORING DEVICE" filed on January 25, 2008; PCT Patent App. No. PCT/US2012/044998 entitled "IMPLANTABLE SENSOR ENCLOSURE WITH THIN SIDEWALLS" filed on June 29, 2012, PCT Patent App. No. PCT/US2011/045581 entitled TRANSVASCULAR WIRELESS SENSOR SYSTEM" filed on July 27, 2011; U.S. Patent App. No. 15/958,613 entitled "ANCHORING SYSTEM FOR A CATHETER DELIVERED DEVICE," filed on April 20, 2018; U.S. Provisional Application No. 62/624,146 entitled "DEVICE AND METHOD FOR DEPLOYING AND SECURING AN IMPLANT TO A VESSEL WALL," filed on January 31, 2018, each of which are incorporated by reference. However, this application is not limited to the use with these devices or assemblies as others may be contemplated.

Steps 10, 20 and 30 are comparable to steps (1), (2) and (3) of Figure 1. Venous access may be established at an access location along the patient's anatomy by puncturing a vein percutaneously 10. This may also include inserting an "introducer" at the access location to support the insertion of various catheters. The femoral vein is a typical access location; jugular, brachial, and subclavian vein are also known access locations. The distal end of a first pulmonary artery catheter ("PAC") is then inserted at the access location 20. A balloon may be inflated along a distal portion or distal top of the PAC within the vascularity of the patient 30.

Notably, step (4) of Figure 1 is not necessary for the process of Figure 3, this is because a contrast map for future use is not appropriate as the anatomy is to be mapped in real time when the implant 101 is near the target site. Here, the PAC includes thee balloon function, so any balloon catheter that can get you to the target site is acceptable - it does not need the additional contrast dye function.

Steps 40, 50, and 60, are similar to steps (5), (6), and (7) of Figure 1. Here, the distal end of the PAC may be located at or near the target site and a guide wire (GW) may be inserted or positioned within a lumen of the PAC 40. The guide wire (GW) may slide or otherwise be withdrawn from a lumen within the PAC to be positioned at or adjacent the target site 50. The PAC may be removed allowing the guide wire (GW) to remain positioned therein. The guide wire (GW) may remain located within the vasculature between the access location and the target site to guide other catheters in and out to the target site 60. Notably different from step (7) is that the instant delivery system with sensor 200 are advanced over the guide wire (GW) to the target site.

The delivery system 200 allows for contrast dye to be injected therefrom to allow for a real-time imaging (fluoroscopy imaging) of the location of the delivery system 200 and implant 101 or sensor relative to the anatomy of the patient 70. This allows for the injection of contrast dye to be performed while the implant 101 is actually near the target site. Further, the radio-opaque markings 205 provide imaging cues to allow the medical practitioner to view and confirm exactly where the delivery system 200 and implant 101 are positioned and adjusted relative to the actual target site or other benchmark locations within the anatomy with live fluoroscopic imaging (steps 80 and 90). This method does not require the medical practitioner to compare the relative position of a catheter viewed in the fluoroscopic image with a static angiogram image acquired when contrast dye was introduced by the PAC moments ago. This step reduces the guess work or estimating of sizes and locations using the static image of the angiogram in the estimation of placing the sensor or implant at the target site in the anatomy.

These steps do not require the use of an anatomic reference marker like ribs, spine, or other implants to estimate where the implant 101 may be positioned or adjusted relative to the target site. This process improves accuracy and reduces the risk of releasing the implant or sensor in an incorrect location (which could be a safety problem). This method may also benefit when the distal anchor and/or proximal anchor include shapes as disclosed by U.S. Patent App. No. 15/958,613 wherein at least one anchor includes an elongated and angled orientation relative to the implant 101 or the at least one anchor may include a clover-shaped structure. This real-time angiography feature, and the configuration of the anchor's relative to the implant 101 may allow a medical practitioner to confidently move and adjust the implant 101 to the best available location in the anatomy prior to its placement. Once the implant is positioned at the target site 80 and adjusted in the best location 90, the implant 101 or sensor may be deployed 100. The implant 101 may be deployed by the medical professional by pulling the release wires 212 thereby retracting the release wires 212 from the distal anchor and the proximal anchor to allow the anchors to expand and anchor the implant in place within the target site.

The inner sheath 202 may be removed from or partially retracted relative to the outer sheath 204 and the anatomy of the body 110. Optionally, the guide wire (GW) may also be removed, it may remain in place, or may be partially removed to provide some stability for the outer sheath 204. A calibration of the implant 101 may be performed 120. Here, a port 230 positioned along a proximal portion (outside the body) is connected to a pressure transducer, so that is it fluidically coupled to the bloodstream, at the distal portion of the second sheath 204. The pressure in the blood vessel is communicated to the port 230 and pressure transducer to provide an accurate reference pressure measurement. Notably, a second catheter is not needed (as is required in the Figure 1 method). The calibration may be performed by comparing a pressure measurement reading taken by the pressure transducer with a pressure measurement reading taken by the implant 101 deployed at the target site. The pressure measurement reading taken by the implant 101 may be wirelessly communicated to a receiver or reader device. One example of a reader device is disclosed by U.S. Patent No. 8,493,187 and its related patents. The deployed implant 101 may be adjusted by mathematically changing calibration coefficients or by using a lookup table and programming or adjusting the reader device or implant 101. The outer sheath 204 may then be removed from the anatomy 130 and the access location may be closed 140.

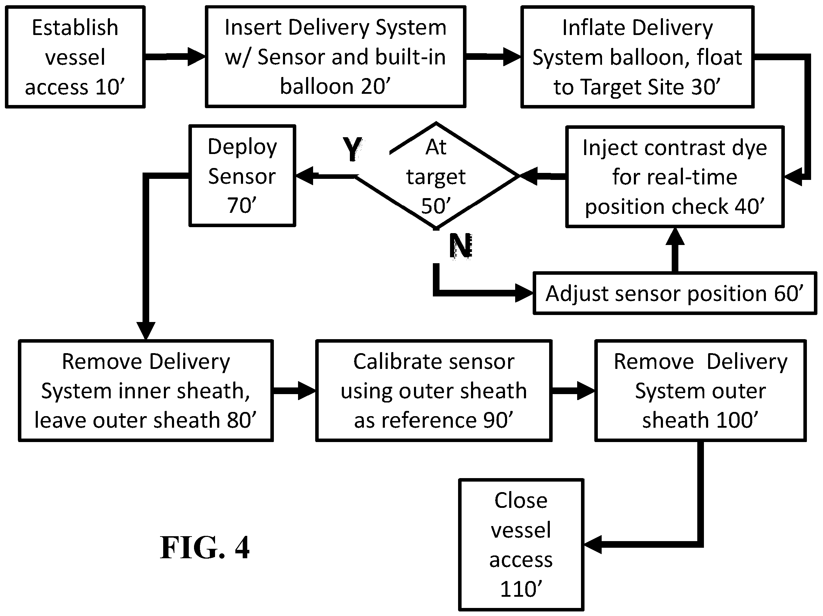

Figure 4 illustrates another embodiment and assumes the use of a different type of delivery system catheter than the type described in Figures 1 or 3. This catheter includes a two-sheath concept as in Figure 2, but also includes the balloon member 220 positioned along a distal portion of the inner sheath 202. This method reduces the need for utilizing a PAC to position the guide wire at the target site of steps (2) and (3) or steps 20 and 30 or to otherwise carry out the procedure.

Step 10' is comparable to step (1) of Figure 1 and step 10 of Figure 3. However, after venous access is established at the access location along the patient's anatomy (10'). The distal end of a delivery system 200 with balloon member 220 and implant 101 is then inserted at the access location (20'). The balloon may be inflated along a distal portion or distal top of the delivery system 200, preferably along the inner sheath 202 within the vascularity of the patient (30'). In this embodiment, there is no need for a guide wire (GW).

Steps 40', 50', and 60', are similar to steps 70, 80, and 80 of Figure 3. The delivery system 200 allows for contrast dye to be injected therefrom to allow for a real-time imaging (fluoroscopy imaging) of the location of the delivery system 200 and implant 101 or sensor relative to the anatomy of the patient 40'. This allows for the injection of contrast dye to be performed while the implant 101 is actually near the target site. Further, the radio-opaque markings 205 provide imaging cues to allow the medical practitioner to view and confirm exactly where the delivery system 200 and implant 101 are positioned and adjusted relative to the actual target site or other benchmark locations within the anatomy with live fluoroscopic imaging (steps 50' and 60'). The remaining steps 70' - 110' are comparable to steps 100-140 of Figure 3.

Once the implant is positioned at the target site 50' and adjusted in the best location 60', the implant 101 or sensor may be deployed 70'. The implant 101 may be deployed by the medical professional by pulling the release wires 212 thereby retracting the release wires 212 from the distal anchor and the proximal anchor to allow the anchors to expand and anchor the implant in place within the target site.

The inner sheath 202 may be removed from or partially retracted relative to the outer sheath 204 and the anatomy of the body 80'. A calibration of the implant 101 may be performed 90'. The outer sheath 204 may then be removed from the anatomy 100' and the access location may be closed 110'.

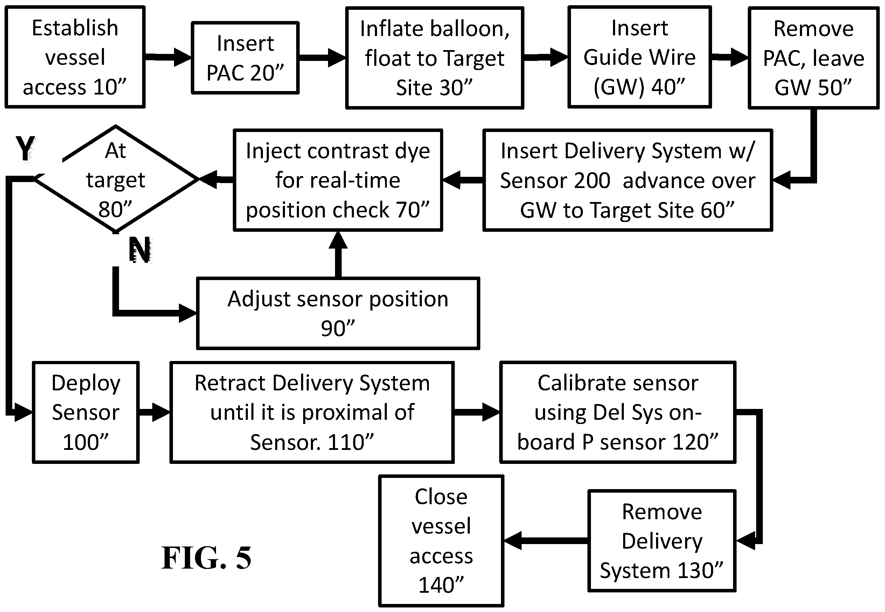

Figure 5 illustrates another embodiment and assumes the use of a different type of delivery system catheter than the type described in Figures 1, 3, and 4. Here, this delivery system 200 includes a two-sheath concept as in Figure 2, but instead of the pressure transducer and the balloon member 220, a reference sensor 210 may be positioned along a distal portion of the outer sheath 204. Steps 10"-140" are otherwise comparable to steps 10-140 of Figure 3. Figure 2 illustrates use of the reference sensor 210 (separate from the implant 101) that is attached along a distal portion of the outer sheath 204. The reference sensor 210 may be powered by a battery and operate wirelessly to communicate pressure data to an outside receiver. Optionally, the reference sensor 210 may be connected to circuitry at the proximal end of the delivery system 200 by contact wires. In this embodiment, the reference sensor 210 is utilized in lieu of the pressure transducer and associated fluid port 230 of the outer sheath 204 (of Figure 3) is not needed to perform the calibration step. Calibration step 120" may thus be performed by the reference sensor 210 that is configured to wirelessly communicate information to a receiver or reader device. This disclosure also contemplates an embodiment of the delivery system 200 that includes the balloon member 220 as well as the reference sensor 210 in which the featured steps of the self-placement of the delivery system 200 with balloon member 200 of Figure 4 may be combined with the reference sensor 210 based calibration of Figure 5.

Further, Figure 6A illustrates an implant 101 from Figure 22 of commonly owned US Patent No 10,206,592 in which distal and proximal anchors 103, 102 are positioned in an expanded configuration within the vasculature of a patient's anatomy. The anchors are disclosed to extend from opposing ends of the implant 101 and are attached to the implant 101 at attachment portions 122. Figure 6A illustrates the implant 101 includes an inner facing surface 132 configured to face inwardly of the vasculature and an opposite outer facing surface 142 configured to abut against the inner wall of the anatomy. Figure 6B is a cross sectional view of the implant 101 according to embodiments of the instant application that illustrates a novel way to configure the attachment portions 122.

The markers 205, discussed above, may be radio opaque to assist medical practitioners to identify a location of a device during fluoroscopy (i.e., x-ray imaging). The markers may be made from radio-opaque chemical compounds which can be added to polymers, ceramics, plastics, or other materials for molding into various shapes. There also exists radio-opaque coatings or inks that can be applied to surfaces. A common product for use in catheters is radio-opaque tubing, such as supplied by Zeus, Polyzen, or Fluortek Corporation.

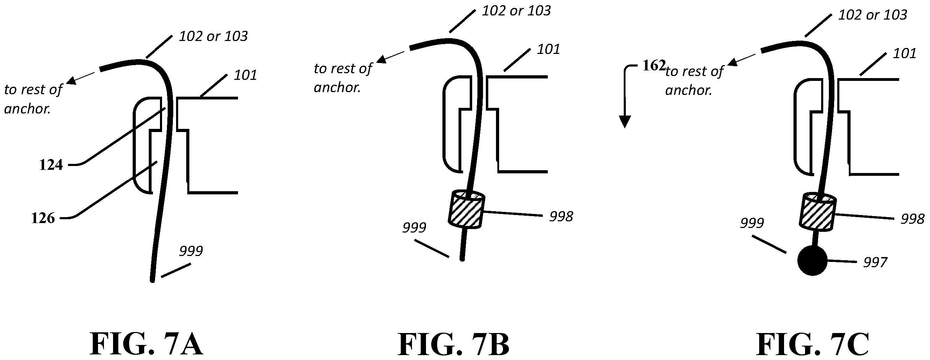

Figures 7A - 7E illustrate an embodiment of the instant disclosure that identifies an improvement over known embodiments of implants that are configured to include markers thereon. In an embodiment of the disclosure, the markers may include a cylindrical shape length of a marker or tube 998 having radio-opaque material thereon. The marker 998 may be a tube cut into a cylinder shape of desired length and attached to a portion of the anchor by a heatshrink, adhesive, or other process such as friction fit.

This disclosure illustrates an improved way to attach and configure markers to assist in fluoroscopic imaging to an implant 101. In an embodiment, a plurality of through holes may be drilled through portions of the implant 101. There may be any number of through holes but in the disclosed embodiment, four holes are provided to include four attachment points 122 to the implant for use with the distal and proximal anchors 103, 102. The through holes may be counter-bored having two overlapping holes of different diameters. A first hole 124 being a through hole having a first size, and a second hole 126 only extending partially through the implant body and having a second size. The first hole 124 may be concentric relative to the second hole 126. In this embodiment, the first size may be smaller than the second size thereby creating a shoulder or stop between the first hole 124 and second hole 126. In Figure 7A, a terminal end 999 of anchor wire 102 or 103 may be threaded through the first and second holes 124, 126, with the terminal end exiting through the large sized second hole 126. Figure 7B illustrates the marker 998 as a tube attached along the anchor wire adjacent to a terminal end of the anchor. The marker 998 may be a cylindrical shaped radio opaque marker tube 998 that is slid over the free end of the anchor. The marker 998 may sit freely along the wire or may be adhered, heat shrunk, crimping, or friction fit thereon. Note that the marker may be any shape and is not limited to being cylindrical.

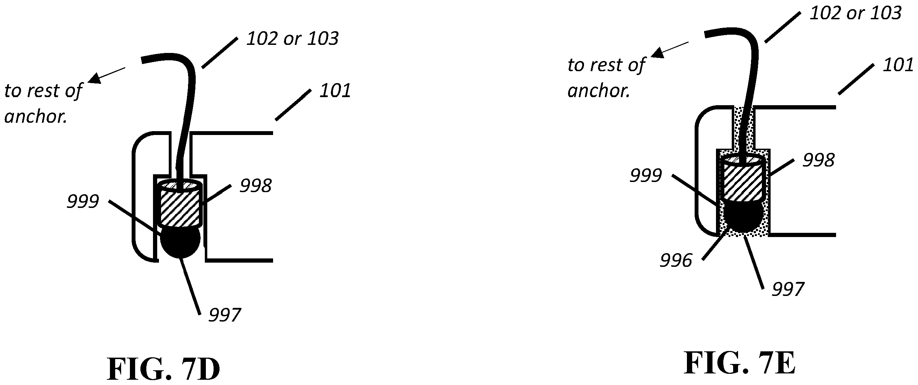

Figure 7C illustrates an enlarged end 997 positioned on the terminal end 999 of the wire of the anchor 102/103. The enlarged end 997 and marker 998 are configured to be positioned within the larger sized second hole 126 while the remaining portion of the anchor extend through the smaller sized first hole 124. As such, at least the enlarged end 997 may abut against the shoulder and may not exit through the small sized first hole 124. The enlarged end 997 may be formed by: heating the end to form a ball; using a laser; stamping; "coining" or otherwise compressing the end to flatten it; bending the end to form a coil shape, or otherwise enlarging it by means known to those skilled in the art. Figure 7D illustrates the assembly having larger materials fit snugly into the large size second hole 126. The enlarged end 997 ensures that the wire cannot exit the first hole 124, increasing system safety and reliability. In Figure 7E, the remaining space in the holes 124, 126 may be filled with a filler material 996, typically adhesive, potting material, epoxy or other filler material that may be safe for human implantation. As implant 101 may be made of a glass or ceramic material, the concentric through hole configuration is not known to be used in an implant assembly, method of manufacture, or delivery system. The embodiments of the marker and implant 101 assembly may include various alternate designs including: (i) without a marker band 998, but use of a radio opaque filler material 996; (ii) no marker band; (iii) crimp-on marker band 998 and no enlarged end (the crimp-on holds the wire in place); (iv) no enlarged end, filler material holds the assembly in place. Notably, two ends of said wire anchor may be attached to said implant body such that said anchor forms a loop and the counter bored hole may be filled with a filler material to prevent translational or rotational movement of said anchor.

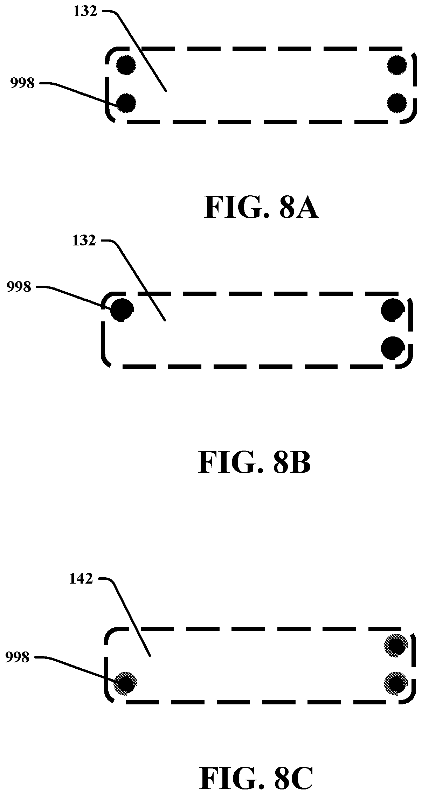

Figures 8A, 8B, and 8C illustrate the concept of providing symmetrical or asymmetrical radio opaque markings. As can be seen in Figure 6A, the implant body, shown in side view, includes an inner facing surface 132 configured to face inwardly of the vasculature and an opposite outer facing surface 142 configured to abut against the inner wall of the anatomy. The inner facing surface 132 may include a sensitive diaphragm desirable to keep away from a vessel wall and facing towards the bloodstream to be able to accurately sense or measure pressure. Figure 8A illustrates markers 998 placed in four corners to allow a fluoroscope image to view the implant 101 to include four dots. The hashed outline of the implant body is for reference only and would likely not be visible on the fluoroscope image. The plurality of said markers comprise an asymmetric pattern on said implant body, said pattern configured to facilitate determination of implant orientation when viewed on a fluoroscope. Said asymmetric pattern may include three marks positioned along three corners of a two-dimensional rectangle when viewed on a fluoroscope.

By making the dot pattern asymmetrical, as in Figure 8B, the fluoroscope image can identify which surface (inner facing surface 132 or outer facing surface 142) and relative position of the implant 101 may be viewable. In an embodiment, the asymmetrical pattern includes placing markers 998 in three of the four corners. In this way, the medical practitioner may easily identify which surface is facing an imaging device to allow for precise adjustment and positioning the implant at the target site in the anatomy. Notably, any type of asymmetrical or symmetrical configuration is contemplated by this disclosure and is not limited to the placement or configuration of markers 998 illustrated by Figures 8A, 8B, and 8C.

Figure 2 illustrates various locations to place markers 205 on either or both the implant 101 and the sheaths 202, 204 of the delivery system 200. These locations include but are not limited to the distal tip 201; distal end of the second sheath 202; a set of ruled stripes along first sheath 204 or second sheath 202. The ruled stripes could be evenly spaced or unevenly spaced. These marker arrangements can help serve as a ruler to judge distances, positions, and adjustments during placement of the implant by viewing live images on the fluoroscope.

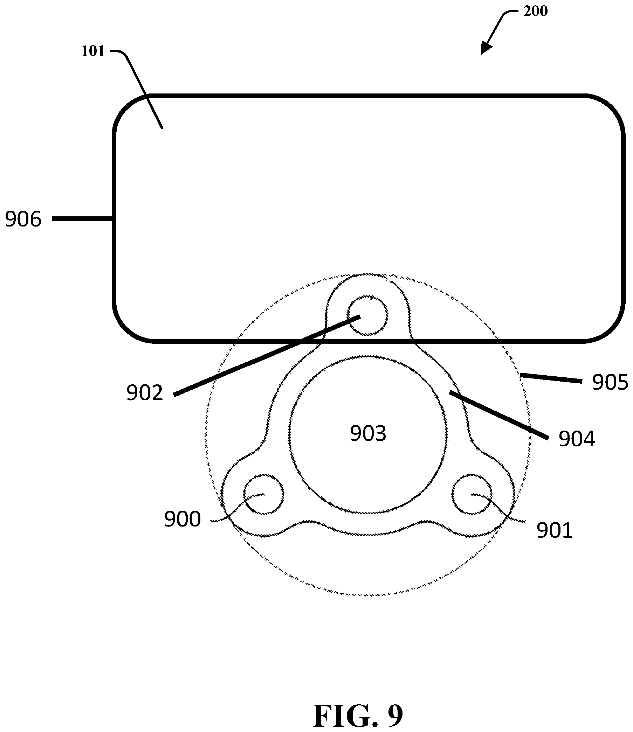

Figure 9 illustrates an embodiment of the delivery system 200 in cross section having the implant 101 mounted thereon as viewed towards the distal end. The inner sheath 202 may include a plurality of lumens aligned along its length. In one embodiment, the inner sheath 202 includes general triangle shape cross section with a plurality of outer lumens 900, 901, and 902 spaced from one another at about 0°, 120°, and 240° and extruded along its length. A center lumen 903 may be positioned along centrally from the outer lumens. Any number of lumens, including one, two, three, four, five, six, seven, or more are contemplated by this disclosure. Each of the plurality of lumens may extend the entire length of inner sheath 202. Additionally, one or more of the lumens may include slots or flattened portions to assist with attachment of the implant 101 thereto.

The hashed line represents an outer perimeter of the outer sheath 204. Part of the inner sheath 202 may be contained inside the outer sheath 204 (not shown except as hashed line), and part of the inner sheath 202 may extend outside the outer sheath 204 as illustrated by Figure 2. The large rectangle 906 represents a body of the implant 101. The body 906 may rest on a flattened region (as a portion of lumen 902 may be cut or flattened out) on the portion of inner sheath 202 that extends outside of outer sheath 204. The center lumen 903 may be larger than the outer lumens 900, 901, 902 and may be configured to support the guide wire (GW) as described above.

Lumens 900 and 901, positioned along the underside of the inner sheath 202 may be to support the release wires 212. In one embodiment, the anchors 102, 103 may be folded down in a collapsed configuration and positioned partially around or tucked under the bottom of the inner sheath 202. The release wires 212 may be made of nitinol, stainless steel, or another extrudable material, and may be contained in lumens 900 and 901. The release wires 212 may be threaded through various slots 215 as identified in Figure 2 to hold the anchors 102, 103 in the collapsed configuration. In an embodiment, the release wires 212 may be pulled proximally from the proximal end of the delivery system (where there may be a handle). As ends of the release wires 212 move past each collapsed anchor 102, 103, the anchor is released and deploys to its unfolded or expanded shape. The anchors may each deploy one at a time or simultaneously.

Alternatively, the delivery system 200 may include a single continuous release wire 212 that aligns through a first lumen (e.g., 900), ties down anchors 102, 103 along a first side of the implant 101 and loops around the distal portion to tie down opposing sides of the anchors 102, 103 on the other side through a second lumen (e.g., 901). Here, the medical practitioner would pull just one end of that release wire 212 to release the anchors 102, 103 from the delivery system 200.

In one embodiment, lumen 902 is not used. In another embodiment, lumen 902 could be used for a third release wire that could support along a top side of implant body 906 to provide more stability and tie-down security. In another embodiment, lumen 902 could be used as a fluid column to connect to the pressure transducer and fluid port 230 located outside the body. Notably, the pressure transducer and fluid port 230 may be positioned along the inner sheath 202 or the outer sheath 204 but by using the inner sheath 202, a medical practitioner may be able to measure a pressure reading during the time the inner sheath 202 is within the body. In another embodiment, lumen 902 may be used to inject fluids such as contrast dye or drugs. Alternatively, the outer sheath 204 can be used to inject fluids such as contrast dye or drugs.

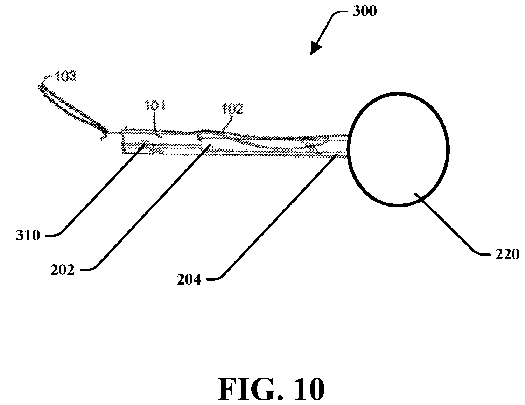

In another embodiment as illustrated by Figure 10, provided is an implant retrieval system 300 for removing an implanted device 100 from a human body. The implanted device having at least one portion (i.e., anchors 102, 103) that may be collapsible and wherein the first sheath 202 and second sheath 204 each extending from a proximal end and at least said first sheath extends to a distal end of the retrieval system 300. The first sheath 202 is positioned at least partially within said second sheath 204 wherein said first sheath 202 includes a linkage member 310 is configured to mechanically couple to a portion of said implanted device 101. The first sheath 202 and said second sheath 204 may be rotatably and translatable with respect to one another and said collapsible anchors 102, 103 of said implanted may be configured to return to a collapsed state when said first sheath 202 or said linkage member 310 retracts said implant 101 proximally into said second sheath 204.

The second sheath 204 of the retrieval system 300 may be configured to hold said collapsible portion 102, 103 of said implant 101 in said collapsed state while said system and said implant are withdrawn from said body. The retraction of said implant 101 may be accomplished by applying distally directed force to said second sheath 204 while applying a proximally directed force to said first sheath 202 or said linkage member 310 while said first sheath 202 is mechanically coupled to said implanted device 101. The retrieval system 300 may also include a balloon member 220 wherein said balloon member 220 may be configured to facilitate reducing blood flow during mechanical capture and retraction of said implanted device. The balloon member 220 may be configured to follow blood flow to initially guide said system to the location of said implanted device prior to retrieval.

The second sheath 204 (of either the retrieval system 300 or the delivery system 200) may be further configured to remain in place to allow exchanging the first sheath 202 for other catheter devices wherein said catheter device includes at least one of: a camera, a pressure sensing catheter, a stent placement device, a valve placement device, a microphone, an ablation device, a balloon device, a Swan Ganz catheter, an electrical stimulation device, an ultrasound device, a drug delivery device, a catheter for gripping implanted devices (linkage member 310), a catheter for readjusting the position of implanted devices, a catheter for removing implanted devices. The second sheath may also be configured to contain a plurality of sheaths in addition to said first sheath. The system 300 may also be configured to detach said implanted device 101 from surrounding tissue while said first sheath is mechanically coupled to said device wherein said detachment of said device from said surrounding tissue comprises cutting off a portion of said device and leaving said portion attached to said tissue as said device is retracted. Alternatively, the detachment of the implanted device from said surrounding tissue may include cutting off a portion of said surrounding tissue and leaving said portion attached to said device as said device is retracted. The detachment may be accomplished by one of: mechanical cutting, tissue ablation, local application of heat, local application of laser energy, local application of radio frequency (RF) energy, local application of ultrasonic energy, local application of vibrational energy.

Having described preferred embodiments of new and improved delivery system and implant configurations and methods, it is believed that other modifications, variations and changes will be suggested to those skilled in the art in view of the teachings set forth herein. It is therefore to be understood that all such variations, modifications and changes are believed to fall within the scope of the present application.

Although the embodiments of the present teachings have been illustrated in the accompanying drawings and described in the foregoing detailed description, it is to be understood that the present teachings are not to be limited to just the embodiments disclosed, but that the present teachings described herein are capable of numerous rearrangements, modifications and substitutions without departing from the scope of the claims hereafter. The claims as follows are intended to include all modifications and alterations insofar as they come within the scope of the claims or the equivalent thereof.

THE FOLLOWING ITEMS DESCRIBE FURTHER ASPECTS, FEATURES AND EMBODIMENTS OF THE PRESENT DISCLOSURE:
1. An implant delivery system comprising:
   an implant;
   a first sheath and a second sheath each extending from a proximal end of said implant delivery system, wherein at least said first sheath extends to a distal end of said implant delivery system, wherein said first sheath is positioned at least partially within said second sheath, the first sheath is translatable relative to said second sheath;
   wherein said implant is connected to an exterior surface of said first sheath, and
   wherein said first sheath and said second sheath are movable with respect to one another to deploy said implant to a target site in an anatomy;
   and further wherein said delivery system is configured to be partially inserted into a living body such that said proximal end remains external to said body and said distal end is internal to said body.
2. The implant delivery system of item 1, wherein said delivery system is configured to be partially inserted into a blood vessel of a human body such that said proximal end remains external to said body and said distal end is internal to said body.
3. The implant delivery system of item 1, further comprising at least one fluid port positioned along a proximal end of said first sheath or second sheath, said fluid port fluidly coupled to at least one lumen extending down the length of said first sheath or second sheath to allow fluid flow through said lumen.
4. The implant delivery system of item 3, wherein said fluid is injected through said port and includes one of: a drug; a fluid used to enhance anatomical imaging; fluoroscopic contrast dye; barium; a radioactive material; blood; plasma; saline solution; a blood component; a particle suspension; a nano-device; and a nanomaterial.
5. The implant delivery system of item 3, wherein said at least one fluid port is further configured to operatively couple to a device located outside of said body.
6. The implant delivery system of item 5, wherein said device is a pressure transducer, configured to measure a fluid pressure at the distal end of said first or said second sheath.
7. The implant delivery system of item 6, wherein said measurement of fluid pressure at the distal end of said first or second sheath is used to calibrate or assess the accuracy of said implant.
8. The implant delivery system of item 1, further comprising at least one marker configured to be visible with a fluoroscope.
9. The implant delivery system of item 8, wherein said marker include a radio opaque material positioned on at least one of: a distal tip of said first sheath; the distal portion of said second sheath; a portion of said implant; and as a plurality of markings spaced along a portion of said first or said second sheath.
10. The implant delivery system of item 8, wherein said marker is attached to at least one anchor on the implant.
11. The implant delivery system of item 8, wherein a plurality of markers are positioned along said implant in an asymmetric pattern, said pattern is configured to facilitate determination of implant orientation when viewed on a fluoroscope.
12. The implant delivery system of item 11 wherein said asymmetric pattern comprises markers at three of the four corners of a two-dimensional rectangle when viewed normal to the plane of said rectangle on a fluoroscope.
13. The implant delivery system of item 1, wherein:
   said second sheath is configured to allow insertion of a catheter device configured to selectively attach to said implant and move the implant proximally when retracted;
   wherein at least one anchor of said implant is collapsible; and
   wherein said anchor of said implant is configured to be placed in a collapsed state when said catheter device moves the implant proximally into said second sheath.
14. The implant delivery system of item 1, wherein said second sheath is further configured to allow said first sheath to be retracted into said second sheath while said implant is still connected to said first sheath, and further configured to cover said first sheath and said implant while said delivery system is retracted and withdrawn from said body.
15. The implant delivery system of item 1, wherein said first sheath is configured with a distal tip made of a soft material to minimize vessel trauma during use, wherein said distal tip has a durometer softer than Shore 40A.
16. The implant delivery system of item 1, wherein said implant further comprises a rigid housing, and at least one collapsible implant anchor, said at least one anchor being attached to said housing.
17. The implant delivery system of item 16, wherein said anchor is comprised of at least one wire that passes through at least one hole that extends through the thickness of said housing; and an enlarged portion of the wire is placed along said wire to retain the wire within the hole; and
   wherein said hole is counter-bored, said counter-bored hole comprising a first size hole and a second size hole, wherein the first size is smaller than the second size, wherein the second hole extends only partially through said housing, and further wherein said enlarged portion is configured to fit into said second hole.
18. The implant delivery system of item 17, wherein said implant includes a marker configured to be at least partially visible under fluoroscopic imaging.
19. The implant delivery system of item 18, wherein a plurality of said markers are spaced at known intervals along said anchor wire to facilitate distance estimation during angiographic imaging; and wherein said marker is located on the anchor wire near a point where said anchor wire attaches to said housing.
20. A method for implanting an implant device in the vasculature of a human body, said method comprising the steps of:
   establishing access to the vasculature at an access location along the patient's anatomy;
   inserting a first catheter configured to translate from the access location to a target site within the vasculature;
   placing a guide wire between said access point to said target location;
   removing said first catheter while leaving said guide wire in place;
   inserting a delivery system over said guide wire, said delivery system comprising a first sheath and a second sheath each extending from a proximal end of said implant delivery system, wherein at least said first sheath extends to a distal end of said implant delivery system and wherein said first sheath is positioned at least partially within said second sheath, and includes an implant attached to said first sheath, wherein said first sheath or second sheath is configured to allow the injection of contrast dye for angiographic imaging;
   advancing said delivery system to said target site while using angiographic imaging to position said implant at said target site;
   deploying said implant from said delivery system at said target site;
   withdrawing said second catheter and said guidewire from said body while said medical device remains at said target location.
21. The method of item 20, wherein said delivery system includes a port configured to remain outside of said vasculature and to fluidically couple a portion of said first sheath or second sheath that is inside said vasculature to a device that is outside of said vasculature, said method further comprising the steps of:
   connecting said port to a pressure measurement device;
   measuring pressure at said inside portion of said first sheath or second sheath using said pressure measurement device;
   measuring pressure at said inside portion with said implant;
   comparing said measurement from said pressure measurement device to a measurement made by said implant.
22. The method of item 21, further comprising calibrating said implant.
23. The method of item 20, wherein said implant is connected to an exterior surface of said first sheath and positioned near a distal end of said second sheath, and wherein said first sheath and said second sheath are movable with respect to one another to deploy said implant to the target site.
24. A method for implanting an implant in a vasculature of a body, said method comprising the steps of:
   establishing access to the vasculature at an access location;
   inserting a catheter device into said vasculature at said access location, said catheter device fitted with said implant and configured to advance to a target site;
   advancing said catheter device to said target site;
   deploying said implant from said catheter device at said target site;
   withdrawing said catheter device from said body while said implant remains at said target site.
25. The method of item 24, wherein said catheter device is configured to inject contrast dye into said vasculature to facilitate angiographic imaging, said method further comprising the step of advancing said catheter to said target location while using angiographic imaging.
26. The method of item 25, wherein said catheter device is configured to fluidically couple a portion of said catheter device inside said vasculature to a portion of said catheter device that is outside of said vasculature, said method further comprising the steps of:
   connecting said portion of said catheter device that is outside of said vasculature to a pressure measurement device;
   before withdrawing said catheter device from said body, measuring pressure at said portion of said catheter device inside said vasculature using said pressure measurement device;
   measuring pressure using said implant;
   comparing said measurement from said pressure measurement device to a measurement made by said implant.
27. The method of item 24, wherein said catheter device comprises a first sheath and a second sheath each extending from a proximal end of said catheter device, wherein at least said first sheath extends to a distal end of said implant delivery system, wherein said first sheath is positioned at least partially within said second sheath;
   wherein said implant is connected to an exterior surface of said first sheath and positioned near a distal end of said second sheath, and
   wherein said first sheath and said second sheath are movable with respect to one another to deploy said implant to a desired target location.
28. A method for attaching a wire anchor to a rigid implant body, comprising the steps of
   providing at least one hole that extends through an implant housing;
   passing an anchor wire through said hole;
   enlarging at least one portion along said wire to prevent said enlarged portion from passing through said hole.
29. The method of item 28 further comprising counter-boring said hole to create a large portion and a small portion.
30. The method of item 29 further comprising the step of placing said enlarged portion of said wire into said large portion of said hole.
31. The method of item 30 further comprising the step of placing at least one preformed fluoroscopic marker over said anchor wire prior to enlarging said dimension of said terminal end.

## Claims

1. An implant delivery system (200) comprising:
an implant (101) configured to measure pressure and to wirelessly communicate information to a receiver or reader device;
a first sheath (202) and a second sheath (204), wherein said first sheath (202) is positioned at least partially within said second sheath (204), the first sheath (202) is translatable relative to said second sheath (204) and wherein the first sheath (202) comprises an exterior circumference;
wherein said implant (101) is directly connected to the exterior circumference of said first sheath (202) and is deployable from the exterior circumference, and
wherein said first sheath (202) and said second sheath (204) are movable with respect to one another to deploy and release said implant (101) to a target site in an anatomy;
and further wherein said first sheath (202) is configured to be partially inserted into a living body such that a proximal end of said second sheath (204) remains external to said living body and a distal end of said first sheath (202) is internal to said living body.

2. The implant delivery system (200) of claim 1, wherein said first sheath (202) is configured to be partially inserted into a blood vessel of said living body such that said proximal end of said second sheath (204) remains external to said living body and said distal end is internal to said living body.

3. The implant delivery system (200) of claim 1, further comprising at least one fluid port (230) positioned along a proximal end of said first sheath (202) or second sheath (204), said fluid port (230) fluidly coupled to at least one lumen extending down a length of said first sheath (202) or second sheath (204) to allow fluid flow through said lumen.

4. The implant delivery system (200) of claim 3, wherein said fluid is injected through said port (230) and includes one of: a drug; a fluid used to enhance anatomical imaging; fluoroscopic contrast dye; barium; a radioactive material; blood; plasma; saline solution; a blood component; a particle suspension; a nano-device; and a nanomaterial.

5. The implant delivery system (200) of claim 3, wherein said at least one fluid port (230) is configured to operatively couple to a device located outside of said body.

6. The implant delivery system (200) of claim 5, wherein said device is a pressure transducer, configured to measure a fluid pressure at the distal end of said first sheath (202) or a distal end of said second sheath (204).

7. The implant delivery system (200) of claim 6, wherein said measurement of fluid pressure at the distal end of said first or second sheath (204) is used to calibrate or assess accuracy of said implant (101).

8. The implant delivery system (200) of claim 1, wherein:
said second sheath (204) is configured to allow insertion of a catheter configured to selectively attach to said implant (101) and move said implant (101) proximally when retracted;
wherein at least one anchor (102, 103) of said implant (101) is collapsible; and
wherein said anchor (102, 103) of said implant (101) is configured to be placed in a collapsed state when said catheter moves the implant (101) proximally into said second sheath (204).

9. The implant delivery system (200) of claim 1, wherein said second sheath (204) is configured to allow said first sheath (202) to be retracted into said second sheath (204) while said implant (101) is still connected to said first sheath (202), and configured to cover said first sheath (202) and said implant (101) while said first (202) and second sheath (204) are withdrawn from said living body.

10. The implant delivery system (200) of claim 1, wherein said first sheath (202) is configured with a distal tip (201) made of a soft material to reduce vessel trauma during use, wherein said distal tip (201) has a durometer softer than Shore 40A.

11. The implant delivery system (200) of claim 1, wherein said implant (101) further comprises a rigid housing, and at least one collapsible implant anchor (102, 103), said at least one anchor (102, 103) being attached to said housing.

12. The implant delivery system (200) of claim 11, wherein said anchor (102, 103) is comprised of at least one wire that passes through at least one hole (124) that extends through the thickness of said housing; and an enlarged end of the wire is placed along said wire to retain the wire within the hole (124); and
wherein said hole is counter-bored, said counter-bored hole comprising a first size hole (124) and a second size hole (126), wherein the first size (124) is smaller than the second size (126), wherein the second hole (126) extends only partially through said housing, and further wherein said enlarged portion is configured to fit into said second hole (126).

13. The implant delivery system (200) of claim 11, wherein said implant (101) includes a marker configured to be at least partially visible under fluoroscopic imaging.

14. The implant delivery system (200) of claim 13, wherein a plurality of said markers are spaced at known intervals along anchor wire (102, 103) of said anchor (102, 103) to facilitate distance estimation during angiographic imaging; and wherein said marker is located on the anchor wire (102, 103) near a point where said anchor wire (102, 103) attaches to said housing.
